# EUROPEAN PATENT APPLICATION

(11) **EP 0 934 737 A1**
(43) Date of publication of application: **11.08.1999**
(21) Application number: 98101941.7
(22) Date of filing: 05.02.1998
(51) Int. Cl.: A61F 13/15

(54) **Absorbent article comprising topsheet with masking capabilities**

(71) Applicant: THE PROCTER & GAMBLE COMPANY, Cincinnati, Ohio 45202 (US)
(72) Inventor: Trombetta, Liberatore Antonio, Silvi (Teramo 64029) (IT)
(74) Representative: Kohol, Sonia

(57) **Abstract**

The invention relates to an absorbent article comprising a topsheet, a backsheet and an absorbent core intermediate the topsheet and the backsheet. The topsheet includes a primary topsheet (10) and a secondary topsheet (20). At least some of the primary topsheet (10) has apertures (16) arranged in a regularly spaced first pattern (14). At least some of the secondary topsheet (20) has indicia (24) arranged in a regularly spaced second pattern (22), the indicia (24) being positioned, shaped and dimensioned to be substantially homogeneously visible through the apertures (16) in a first or non-position, the secondary topsheet (20) being pivoted relative to the primary topsheet (10) by a pivot angle from the first position to a second or in-use position so that the indicia (24) of the secondary topsheet (20) are differentially out of alignment with the apertures (16) of the primary topsheet (10), forming a regularly spaced third pattern (26).

The regularly spaced third pattern (26) facilitates correct positioning of the absorbent article whilst, simultaneously, masking any colored fluid absorbed by the absorbent core.

## Description

The present invention relates to absorbent articles and, in particular, to absorbent articles in the form of disposable diapers, sanitary napkins, panty liners, incontinence products, absorbent wound dressings and the like.

Conventional absorbent articles normally comprise a fluid-pervious topsheet having a user-facing surface, a fluid impervious backsheet having a garment-facing surface and an absorbent core located intermediate the topsheet and the backsheet. Any body fluids such as blood, urine and the like contact the topsheet and are then transmitted through the topsheet to the absorbent core.

It is known in absorbent article art that, as the absorbent article absorbs colored body fluid, the absorbent core changes color to that of the fluid being absorbed. This is distasteful to the user. It is, therefore, extremely desirable to provide a clean appearance and a dry surface after the discharge of colored body fluids thereon.

The problems associated with prior art absorbent articles are overcome by the generation of a regularly spaced third pattern, when a primary topsheet having apertures arranged in a regularly spaced first pattern is pivoted relative to a secondary topsheet having indicia arranged in a regularly spaced second pattern, from a first position in which the indicia are positioned, shaped and dimensioned to be aligned and accommodated within the apertures, to a second position in which the indicia are differentially out of alignment with the apertures.

The regularly spaced third pattern provides the user, before use, with a visually perceived pattern which is appealing to the user. The regularly spaced third pattern also provides the user, after contact with colored body fluids deposited thereon, with an absorbent article whose colored absorbent core is masked or hidden from the user's view.

It is thought that the regularly spaced third pattern is somewhat analogous to an undesirable effect known as a Moiré pattern observed in multi-color half-tone printing. In, for example, four-color printing, the respective half-tone screens are angled at 45°(black), 75° (magenta), 90° (yellow) and 105° (cyan) and a mis-alignment as small as 0.1° between the respective screen angles can cause a serious (and undesired) Moiré pattern.

It has surprisingly been found that a similar-type effect can be achieved with a primary topsheet having apertures arranged in a regularly spaced first pattern and a secondary topsheet having indicia arranged in a regularly spaced second pattern. This is all the more surprising since the beneficial effect of masking or hiding colored fluid in the absorbent core has been achieved by harnessing an undesired effect in an unrelated field of technology.

It will also be appreciated that, if the regularly spaced third pattern overlies the absorbent core and is substantially co-extensive therewith, this serves, in addition, to optimally position the absorbent core for optimal body fluid absorbtion.

According to a first aspect of the present invention there is provided an absorbent article comprising a topsheet having a user-facing surface, a backsheet having a garment-facing surface and an absorbent core intermediate the topsheet and the backsheet, characterised in that the topsheet includes a primary topsheet and a secondary topsheet; at least some of the primary topsheet having apertures arranged in a regularly spaced first pattern; the secondary topsheet being in face-to-face relation to the primary topsheet and overlying the absorbent core; at least some of the secondary topsheet having indicia arranged in a regularly spaced second pattern, the indicia being positioned, shaped and dimensioned to be substantially homogeneously visible through the apertures in a first or non-use position, the secondary topsheet being pivoted relative to the primary topsheet by a pivot angle from the first position to a second or in-use position so that the indicia of the secondary topsheet are differentially visible through the apertures of the primary topsheet, forming a regularly spaced third pattern.

Preferably, the regularly spaced third pattern overlies the absorbent core and is substantially co-extensive therewith.

More preferably, the primary topsheet is a three-dimensional formed film.

Preferably, the pivot angle is not 0°,90°,180°,270° or 360°.

More preferably, the pivot angle is selected from 0.1°-29.9°, 60.1°-89.9°, 90.1°-119.9°, 150.1°-179.9°, 180.1°-209.9°, 240.1°-269.9°, 270.1°-299.9° or 330.1°-359.9°, even more preferably 5°-20°, 70°-85°, 95°-110°, 160°-175°, 185°-200°, 250°-265°, 275°-290° or 340°-355°; even more preferably 7.5°-15°, 75°-82.5°, 97.5°-105°, 165°-172.5°, 187.5°-195°, 255°-262.5°, 277.5°-285° or 345°-352.5°.

It will of course be appreciated that the desired pivot angle is determined by the regularly spaced first and second patterns so that, if the regularly spaced first and second patterns are altered, the desired pivot angle is empirically obtained by pivoting the primary and secondary topsheets relative to each other, observing the formation of a regularly spaced third pattern and recording the pivot angle in question.

Advantageously, the primary topsheet comprises first and second primary top layers, at least some of each of said first and second primary top layers having apertures arranged in regularly spaced fourth and fifth patterns, the regularly spaced first pattern being the overlapping apertures of the regularly spaced fourth and fifth patterns.

More advantageously, the secondary topsheet comprises a first transparent or translucent secondary top layer, at least some of which having indicia arranged in a regularly spaced sixth pattern and an underlying second secondary top layer, at least some of which having indicia arranged in a regularly spaced seventh pattern, the regularly spaced second pattern of indicia being the composite indicia of the regularly spaced sixth and seventh patterns.

Preferably, at least one indicium of the regularly spaced second pattern is positioned, shaped and dimensioned to be substantially homogeneously visible in the first position, through each aperture of the regularly spaced first pattern.

Alternatively, each indicium of the regularly spaced second pattern is positioned, shaped and dimensioned to be substantially homogeneously visible in the first position, through an aperture of the regularly spaced first pattern.

Preferably, the indicia forming the regularly spaced second pattern are substantially homogeneously shaped.

According to a second aspect of the invention there is provided a method of forming a topsheet for an absorbent article, the method comprising the steps of:-
forming a primary topsheet, at least some of said primary topsheet having apertures arranged in a regularly spaced first pattern;
forming a secondary topsheet, at least some of said secondary topsheet having indicia arranged in a regularly spaced second pattern;
pivoting the secondary topsheet relative to the primary topsheet by a pivot angle from a first position, in which the indicia are positioned, shaped and dimensioned to be substantially homogeneously visible through the apertures, to a second position, in which the indicia are differentially visible through the apertures, forming a regularly spaced third pattern;
and fixing the primary and secondary topsheets in the second position.

According to a third aspect of the invention there is provided a method of forming an absorbent article comprising a topsheet, a backsheet and an absorbent core intermediate the topsheet and the backsheet, the method comprising the steps of:
forming the topsheet in accordance with the second aspect of the invention; and providing the absorbent core and the backsheet in association with the topsheet.

While the present invention will be described in the context of sanitary napkins, the invention is not, by any means, limited to such application. To the contrary, the invention may be applied to great advantage in absorbent articles such as diapers and incontinence products.

The term "absorbent article" as used herein embraces articles which absorb and contain body exudates. More specifically, the term relates to articles placed against or in proximity to the body of a user to absorb and contain the various exudates discharged from the user's body. The term "absorbent article" is intended to include diapers, sanitary napkins, panty liners, incontinence products, absorbent wound dressings and any other articles used to absorb body fluids or body exudates.

### TheTopsheet

The topsheet for use in the present invention comprises a primary topsheet formed from at least one primary top layer and a secondary topsheet formed from at least one secondary top layer, the secondary topsheet being in face-to-face relation to the primary topsheet. The secondary topsheet of the topsheet overlies the absorbent core.

The primary topsheet should be compliant, soft feeling, and non-irritating to the wearer's skin. The primary topsheet is fluid pervious, permitting fluids (e.g., menses and/or urine) to readily penetrate through its thickness. A suitable primary topsheet can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred primary topsheets for use in the present invention are selected from high loft nonwoven topsheets and apertured formed film topsheets. Apertured formed films are especially preferred for the primary topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film primary topsheets are disclosed in U.S. Patent 4,609,518 and U.S. Patent 4,629,643. A preferred primary topsheet for the present invention comprises the three-dimensional formed film described in one or more of the above patents and marketed on sanitary napkins by The Procter & Gamble Company of Cincinnati, Ohio as "DRI-WEAVE". WO 97/14388 also describes a suitable apertured primary topsheet.

Preferred primary topsheets have apertures in a regularly spaced first pattern, the apertures being dimensioned to permit show-through (or visualisation) of the regularly spaced second pattern of indicia on the secondary topsheet.

A preferred regularly spaced first pattern of apertures comprises a macroscopically expanded pattern of irregularly shaped pentagonal apertures described in U.S. Patent No. 4,463,045. The primary topsheet described in U.S. Patent No. 4,463,045 is a three-dimensional formed film comprising an array of sub-patterns, each sub-pattern comprising four irregular pentagonal apertures forming an irregular hexagon. Another preferred primary top layer exhibits apertures arranged in an ordered or pseudo-random array. A suitable ordered array may be a regular square, rectangular, rhomboidal or hexagonal array, the apertures themselves being square or, alternatively, circular or slightly elliptical as described in U.S. Patent No. 4,780,352. The interspacing between adjacent apertures may be about 1.4 mm. Overall, the ratio between the surface occupied by the apertures and the whole surface of the primary top layer is typically within the range 10-50%, depending on the requirements of use and the strength characteristics of the materials used. Preferably, the open area is about 25%. It will of course be appreciated that the apertures may be arranged in an array other than a regular square, rectangular, rhomboidal or hexagonal array or other than in a pseudo-random array. Suitable apertures may be made by a perforating or punching action. A suitable perforating station would constitute two counter-rotating rollers, the lower roller of which acts as a rotary support and has a generally smooth surface and the upper roller of which has teeth or projections arranged in the array corresponding to the first pattern. Feeding of the primary top layer through the perforation station causes the teeth or projections of the upper roller to penetrate the primary top layer, thereby perforating its structure, as described in U.S. Patent No. 4,780,352.

Primary topsheets having not a homogeneous distribution of liquid passage ways but having only a portion of the primary topsheet comprising liquid passage ways are also contemplated by the present invention. Typically such primary topsheets would have the liquid passage ways oriented such that they result in a centrally permeable and peripherally impermeable primary topsheet for liquids.

The body surface of the formed film primary topsheet can be hydrophilic so as to help liquid to transfer though the primary topsheet faster than if the body surface was not hydrophilic. In a preferred embodiment, surfactant is incorporated into the polymeric materials of the formed film primary topsheet such as is described in PCT-publication WO 93/09741. Alternatively, the body surface of the primary topsheet can be made hydrophilic by treating it with a surfactant such as is described in U.S. Patent 4,950,254.

Another alternative are so called hybrid primary topsheets which incorporate fibrous and film like structures, particularly useful embodiments of which are disclosed in PCT publications WO 93/09744; WO 93/11725 or WO 93/11726.

The primary topsheet typically extends across the whole of the absorbent structure and outside the area coextensive with the absorbent structure. The primary topsheet can extend and form part or all of the preferred side flaps, side wrapping elements or wings.

When referring to the primary topsheet, a multi layer apertured structure or a mono layer apertured structure are each contemplated. The hybrid primary topsheet mentioned above is such a multi layer design.

It will be appreciated that the regularly spaced third pattern will be altered if the size and/or shape of the apertures is altered and/or if the number of apertures in each sub-pattern, if present, is altered.

The secondary topsheet is fluid pervious, permitting fluids (e.g., menses and/or urine) to readily penetrate through its thickness. A suitable secondary topsheet can be manufactured from a wide range of materials such as woven and non-woven materials. Suitable woven and non-woven materials can be comprised of natural fibers (e.g., wood or cotton fibers), synthetic fibers (e.g. polymeric fibers such as polyester, polypropylene or polyethylene fibers), a combination of natural and synthetic fibers or bi/multi-component fibers.

The color of the selected material affects, particularly, the color reproduction of lighter tints of indicia. It will be appreciated that the indicia are more distinct against a soft (yellowish) white, whilst process colors reproduce most accurately on neutral white material. The brightness of the selected material may be altered, to adjust the contrast or brilliance of the indicia. Color reproduction may be affected if artificial brighteners (e.g., fluorescent additives) are incorporated in the selected material, since most artificial brighteners are not neutral in color but, rather, have excess blue reflectance.

A particularly suitable woven material for use as a secondary topsheet is paper or tissue.

The secondary topsheet receives the indicia in the regularly spaced second pattern in any manner known in the art.

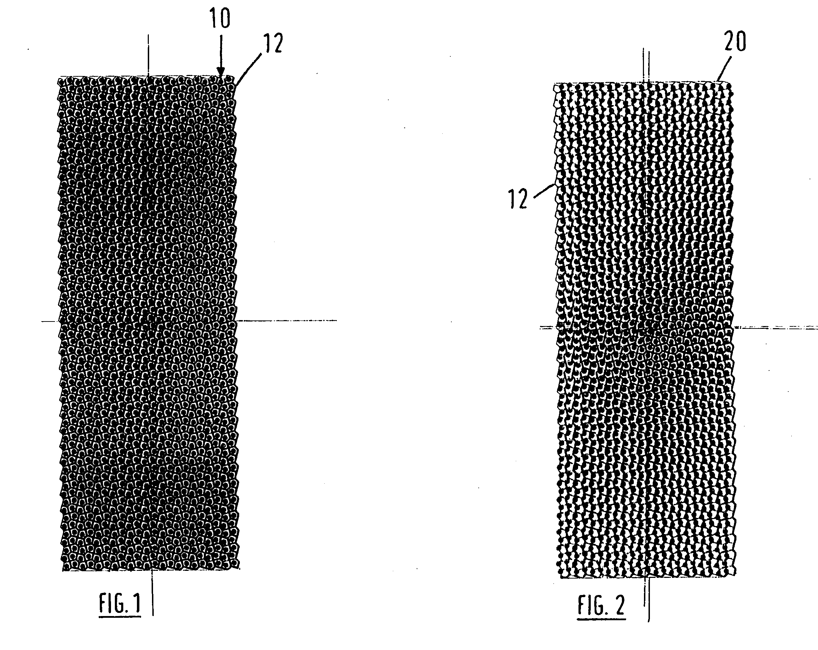

The indicia are positioned in a regularly spaced second pattern. The indicia are aligned and visible through at least some of the apertures of the regularly spaced first pattern in a first position. The indicia may be of various shapes or sizes, colors and/or tones, with different distributions and densities depending on the requirements of use. Thus, for example, one indicium may be positioned, shaped and dimensioned, to be visible in the first position through a respective aperture of the regularly spaced first pattern and, in that event, the regularly spaced first pattern corresponds with the regularly spaced second pattern in the first position. The indicia may be of various tonal intensities with, for example, lighter and darker tonal areas arranged in alternate stripes of varying widths. Although the size of each indicium may exceed the size of the respective apertures in the first position, this is not usually preferred since definition of the resultant third pattern may suffer. Preferably, the size of each indicium is less than or equal to the size of the respective aperture in the first position - as the size of each indicium is reduced, the tonal intensity of the resultant third pattern is also reduced. Alternatively, the indicia may be slightly larger than the size of the respective apertures, for example, 1-25% larger than the size of the apertures for the primary and secondary topsheets of Figures 1-7 described hereinafter.

Further alternatively, the size of the apertures remains constant and the interspacing between adjacent apertures is altered. In this embodiment, if the interspacing between adjacent apertures becomes larger, the size of the indicia can also increase, without affecting the definition of the resultant third pattern. However, as the interspacing increases, without a concomitant increase in the aperture size, the topsheet's ability to rapidly absorb body fluids decreases. Thus, the upper limit on the size of the indicia is limited by a trade-off with the diminished effectiveness of the topsheet.

Alternatively, one indicium is positioned, shaped and dimensioned to be visible through a respective aperture but not all of the apertures accommodate an indicium. This is achieved, by example, by virtue of a regularly spaced second pattern, in which longitudinally extending stripes comprising no indicia are provided on the secondary top layer. Thus, in that event, each indicium is visible through a respective aperture in the first position but there are, in addition, apertures not accommodating indicia.

Alternatively, the indicia may be shaped and dimensioned so that more than one indicium is visible through a respective aperture.

The indicia may be regular or irregular in shape. Suitable shapes of indicia include, but are not limited to, triangles, tetragons, pentagons, hexagons, circles, ellipses, crescent-shapes, teardrops, obround shapes or a mixture thereof.

The regularly spaced second pattern can comprise, alternatively, an irregular column of spaced-apart indicia and an irregular column of adjacent marks forming a wavy line.

The regularly spaced second pattern may be applied onto a secondary topsheet of any one color or any mixture thereof. It will be appreciated that the color choice may have a psychological effect since, for example, red would be an "angry" color whilst blue would be a calming color. If the secondary topsheet is pre-colored, the indicia may be applied in white ink. This gives a reversed, but sharper, impression of the regularly spaced third pattern.

For example, the indicia may be applied by any printing process known in the art, for example, letterpress, lithography, gravure or silk screen. Suitable printing processes are, for example, described in U.S. Patent No. 5,695,855. The regularly spaced second pattern of indicia may be obtained by generating a halftone in any conventional way. Thus, a preferred printing process is half tone printing. As used herein, the term "halftone" means breaking up a continuous solid tone into a plurality of tiny individual indicia of varying sizes, shapes and/or tonal intensities (tonalities).

The smoothness of the selected secondary topsheet material is an important property for letterpress or gravure printing but has little effect on lithography. As the smoothness decreases, halftones get sandy and rough in appearance.

The ink used to form the indicia may be any ink known in the art such as those described in, for example, U.S. Patent No. 5,695,855. The ink used should be safe for human use and should not have environmentally deleterious effects. The ink chosen should, of course, be suitable for the intended printing process. Thus, for example, letterpress and lithographic inks are fairly stiff and require long ink roller trains on the press, to obtain the required flow and film thickness for printing. In contrast, gravure and flexographic inks are very fluid and dry mainly by solvent evaporation. Inks for screen printing are paint-like in their consistency and drying characteristics.

The ink used to form the indicia should be substantially insoluble in the fluids (e.g., menses and/or urine) to be absorbed by the absorbent article.

Alternatively, the secondary topsheet may be embossed with a regularly spaced second pattern of embossing points or "density gradients", following which ink is applied substantially homogeneously across the secondary topsheet and, by capillary force, the ink is drawn to the respective embossing points, thereby providing indicia of varying tonal intensities.

A preferred secondary topsheet for use in the present invention is of a laminate-type structure, in which the primary topsheet-facing surface of a first layer is made up of spun bonded polyethylene non-woven material and the second layer is a combination of cellulose fibers and polyethylene powder. Preferably, the first layer is pre-treated to obtain "embossing points" as described in U.S. Patent No. 4,397,644.

### Absorbent core

Absorbent cores suitable for use in the present invention may be selected from any of the absorbent cores or core systems known in the art. As used herein, the term "absorbent core" refers to any material or multiple material layers whose primary function is to absorb, store and distribute fluid.

The absorbent core for use in the present invention can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### (a) Primary/Secondary Fluid Distribution Layer

One optional component of the absorbent core for use in the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent article. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

### (b) Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", "hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared from polymerizable, unsaturated, acid-containing monomers which are well known in the art.

Suitable carriers include materials which are conventionally utilised in absorbent cores such as natural, modified or synthetic fibers, particularly modified or non-modified cellulose fibers, in the form of fluff and/or tissues. Suitable carriers can be used together with the absorbent gelling material. However, they can also be used alone or in combinations. Most preferred are tissue or tissue laminates in the context of sanitary napkins and panty liners.

One embodiment of an absorbent core for use in the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### (c) Optional Fibrous ("Dusting") Layer

An optional component for inclusion in the absorbent core for use in the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### (d) Other Optional Components of the absorbent core

The absorbent core for use in the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent cores.

Another component which can be included in the absorbent core of the invention and preferably is provided close to or as part of the primary or secondary fluid distribution layer are any odor control agents known in the art.

### Backsheet

The backsheet primarily prevents the exudates absorbed and contained in the absorbent structure from wetting garments that contact the absorbent product such as underpants, pants, pyjamas and undergarments. The backsheet is preferably impervious to fluids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent article and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance.

Preferably the backsheet of the absorbent article is moisture vapour permeable and thus comprises at least one gas permeable layer. Suitable gas permeable layers include two dimensional, planar micro- and macroporous films, macroscopically expanded films, formed apertured films and monolithic films. The apertures in said layer may be of any configuration, but are preferably spherical or oblong and may also be of varying dimensions. The apertures preferably are evenly distributed across the entire surface of the layer. However layers having only certain regions of the surface having apertures are also envisioned.

Suitable two dimensional planar layers of the backsheet may be made of any material known in the art, but are preferably manufactured from commonly available polymeric materials. Suitable materials are for example Gortex (TM) or Sympatex (TM) type materials well known in the art for their application in so-called breathable clothing. Other suitable materials include XMP-1001 of Minnesota Mining and Manufacturing Company, St. Paul, Minnesota, USA and Exxaire XBF-101 W, supplied by the Exxon Chemical Company. As used herein, the term "two dimensional planar layer" refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3,929,135. The apertures located within the plane and the orifices located at the terminating end of protuberances themselves may be circular or non circular provided the cross sectional dimension or area of the orifice at the termination of the protuberance is smaller than the cross sectional dimension or area of the aperture located within the garment facing surface of the layer. Preferably, said apertured performed films are uni-directional such that they have at least substantially, if not complete one directional fluid transport towards the core.

Suitable macroscopically expanded films for use herein include films as described in for example in US Patent No. 4 637 819 and US Patent No. 4 591 523.

Suitable monolithic films include Hytrel™, available from DuPont Corporation, USA, and other such materials as described in Index 93 Congress, Session 7A "Adding value to Nonwovens', J-C. Cardinal and Y. Trouilhet, DuPont de Nemours international S.A, Switzerland such as Pebax™, available from Elf Atochem (France) and Estane™ available from B.F. Goodrich (Belgium).

Particularly preferred backsheets for the present invention comprise at least two layers comprising at least one layer selected from the above, such as microporous and apertured formed films and an additional layer which may also be selected from the above listed backsheets or may be a fibrous woven or nonwoven material. The most preferred breathable backsheet component comprises a microporous film and an apertured formed film or a microporous and a hydrophobic woven or nonwoven material.

Suitable absorbent articles of the present invention include, but not limited to, disposable diapers, sanitary napkins, panty liners, incontinence products, absorbent wound dressings and the like.

A preferred sanitary napkin or panty liner of the present invention has a pair of side wrapping elements or "undergarment covering components". These elements or components provide coverage of the wearer's panties to reduce side soiling (i.e., staining of the edges of the panty crotch) and are typically smaller than conventional flaps or wings.

The function of the side wrapping elements, whether integral with the absorbent article or joined to the absorbent article after being formed separately, is further improved by rendering them extensible in one or both directions, parallel to the longitudinal axis and/or to the lateral axis. The extensibility can be provided across all or only part of the side wrapping elements and can be achieved by pleating or ring-rolling those parts which are to be rendered extensible.

If the absorbent article is a diaper, the diaper may be provided with leg cuffs.

The topsheet, backsheet and absorbent core components are joined together to provide the absorbent article according to the present invention. Typically, at least two, preferably all, of the components are joined to form the absorbent article. Each of said components of the absorbent article comprise at least one layer and have a wearer facing surface and a garment facing surface. Typically, the adjacent garment facing surface forms a common interface with the wearer facing surface of an adjacent component or layer. The elements or layers are joined together across this common interface. In this manner, the topsheet is joined to the absorbent core, and the core is joined to the backsheet. In addition, the topsheet may be directly or indirectly joined to the backsheet at the periphery of the absorbent article. Furthermore, particularly in sanitary napkin, panty liner and incontinence product applications, the garment facing surface of the backsheet also provides the surface to which the absorbent article is releasably joined to the garment of the user of the product. Prior to use, this surface is typically provided with a protective cover. Any means known in the art to join the components of the absorbent article and provide the garment fastening may be utilised e.g., a continuous layer of adhesive, a patterned layer of adhesive, such as spirals, or spots, or using heat bonds, pressure bonds, mechanical bonds and the like.

The invention will now be described, by way of example, with reference to the accompanying drawings, in which:-
Figures 1 - 7 illustrate enlarged views of a primary topsheet and a secondary topsheet at pivot angles of 0°, 1°, 5°, 7.5°, 10°, 20° and 90°, respectively;
Figure 8 - 11 illustrate alternative secondary topsheets; and
Figure 12 illustrates an alternative primary topsheet.

In the drawings, similar numerals have been given to indicate like parts.

Figure 1 illustrates a primary topsheet 10 comprising one primary top layer 12. The primary top layer 12 is a three-dimensional formed film exhibiting a macroscopically expanded three-dimensional first pattern 14 of irregularly shaped pentagonal apertures 16 as described in U.S. Patent No. 4 463 045. Four pentagonal apertures 16 are arranged in a sub-pattern 18 of an irregular hexagonal shape, an array of the sub-patterns 18 forming the regularly spaced first pattern 14.

It is preferred that the maximum dimension of the pentagonal apertures 16 is about 0.889mm (0.035 inches) and that the side walls of said apertures 16 are orientated substantially perpendicular to the plane of the primary top layer 12.

A secondary topsheet 20 is a laminate-type structure, in which the first layer is made up of spun bonded polyethylene non-woven material and the second layer is a combination of cellulose fibers and polyethylene powder. Embossing points are obtained as described in U.S. Patent No. 4 397 644 and ink is then applied, thereby forming a regularly spaced second pattern 22 of substantially round indicia 24. It is preferred that the indicia 24 have a diameter of approximately 0.75 mm, so that the indicia 24, in the first position of Figure 1, almost fill the apertures 16. However, it will be appreciated that the actual shape of each indicium 24 has little effect on the final image since, at this size, the shape of each indicium 24 is almost indiscernible to the human eye.

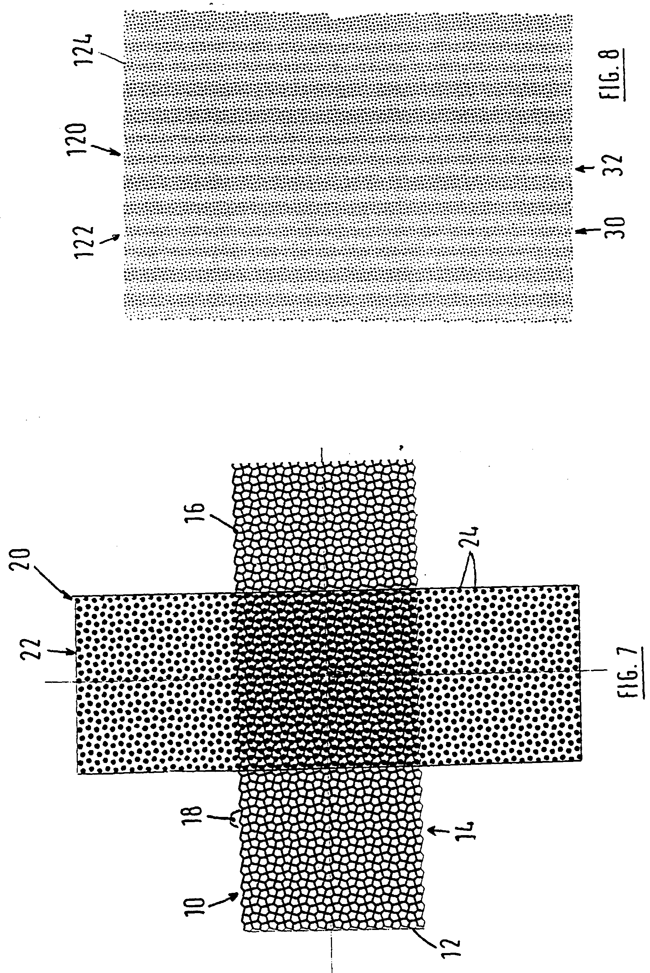

Referring now in particular to Figure 1, it will be observed that each indicium 24 is positioned, shaped and dimensioned to be visible through and, more specifically, to be aligned and accommodated within, a respective aperture 16. Thus, at a pivot angle of 0° (see Figure 1) and, indeed, at a pivot angle of 90° (see Figure 7), 180° (not shown) and 270° (not shown), the indicia 24 are substantially homogeneously visible in the apertures 16, so that a regularly spaced third pattern is not formed. In Figure 1 (first or non-use position), the indicia 24 almost fill all of the apertures 16, thereby creating a substantially homogeneous dark image and in Figure 7, the indicia 24 only partly fill the apertures 16, thereby creating a substantially homogeneous lighter image.

The first pattern 14 of apertures 16 may be present on substantially all of the primary topsheet 10 or, alternatively, may be localised in an area overlying and co-extensive with the absorbent core (not shown). Similarly, the second pattern 22 of indicia 24 may be present on substantially all of the secondary topsheet 20 or, alternatively, may be localised in an area overlying and co-extensive with the absorbent core (not shown). It will, of course, be appreciated that the third pattern 26 will only be visible where the first and second patterns 14,22 are in overlapping, face-to-face relation and where the first and second patterns 14,22 are pivoted relative to each other, to yield the third pattern 26, as will be described hereinunder.

Referring now to Figure 2 of the accompanying drawings, there is illustrated a secondary topsheet 20 which is pivoted at an angle of 1° relative to the primary topsheet 10. A regularly spaced third pattern 26 of darker images or clusters 28 is formed - a single darker image or "cluster" 28 is visible in Figure 2.

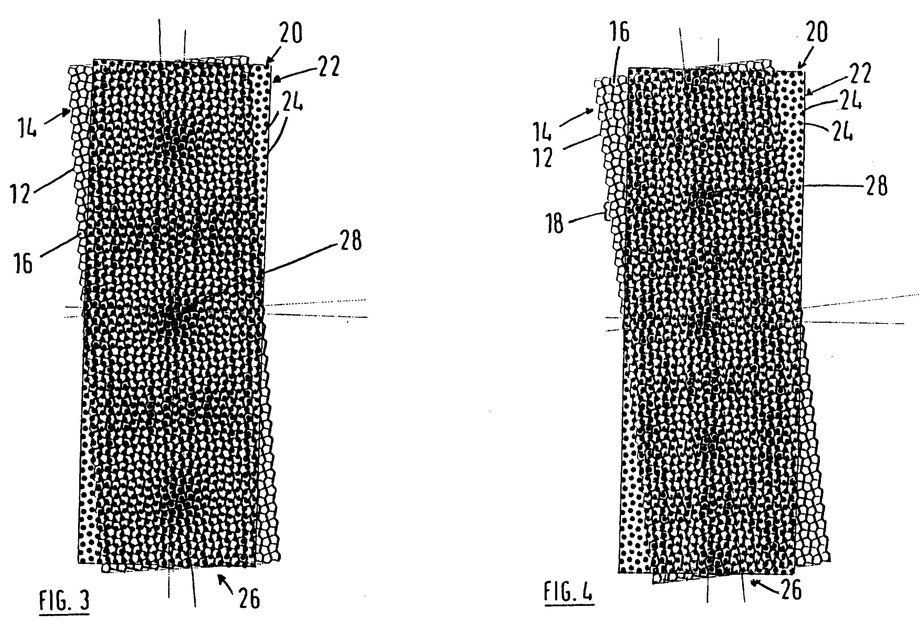

Referring now to Figure 3, there is a pivot angle of 5° between the primary top layer 12 and the secondary topsheet 20. A regularly spaced third pattern 26 of darker images or clusters 28 is formed - three darker images or clusters 28 are visible in Figure 3.

Referring now to Figure 4, there is a 7.5° pivot angle between the primary top layer 12 and the secondary topsheet 20. A regularly spaced third pattern 26 of darker images or clusters 28 is formed - five darker images or clusters 28 are visible in Figure 4.

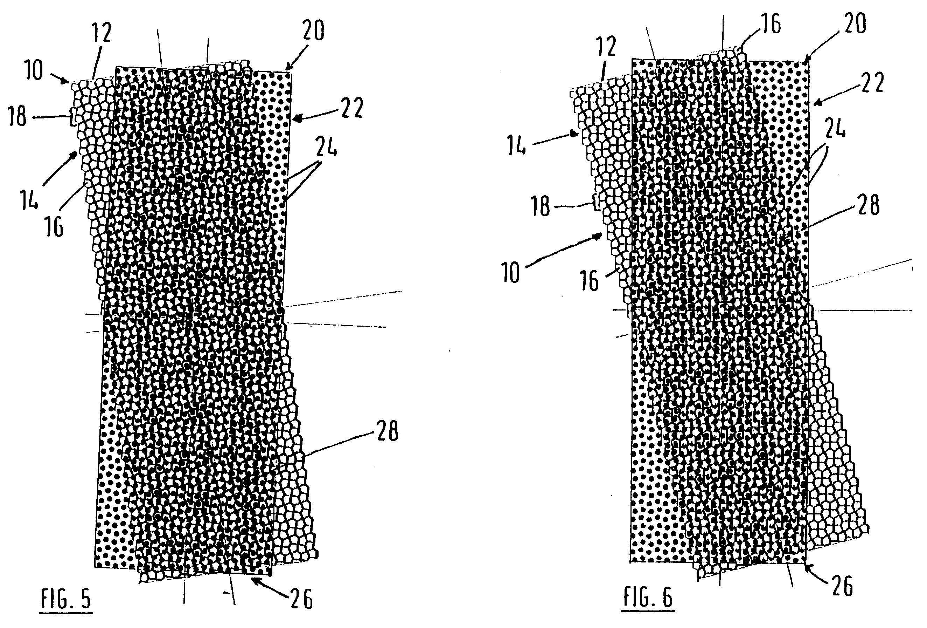

Referring now to Figure 5, the pivot angle is 10° between the primary top layer 12 and the secondary topsheet 20. A regularly spaced third pattern 26 of darker images or clusters 28 is formed - a plurality of darker images or clusters 28 are visible in Figure 5.

Referring now to Figure 6, the pivot angle is 20° between the primary top layer 12 and the secondary topsheet 20. A regularly spaced third pattern 26 of darker images or clusters 28 is formed - a plurality of darker images or clusters 28 are visible in Figure 6.

It will be appreciated that, whilst Figures 1-7 illustrate a regularly spaced second pattern 22, in which one indicium 24 is visible, in the first position illustrated in Figure 1,in each aperture 16, a regularly spaced third pattern 26 will still be formed if, for example, one indicium 24 is positioned, shaped and dimensioned to be visible in the first position illustrated in Figure 1 in one of the four apertures 16 forming each sub-pattern 18. This will, of course, result in a regularly spaced third pattern 26 of lower tonal intensity and lower image definition.

Referring to Figure 8 of the accompanying drawings, there is illustrated a second embodiment of a secondary topsheet 120 having a regularly spaced second pattern 122 of indicia 124, in which the tonal intensity of the indicia 124 is formed from alternate stripes 30, 32 of darker and lighter indicia 124, respectively.

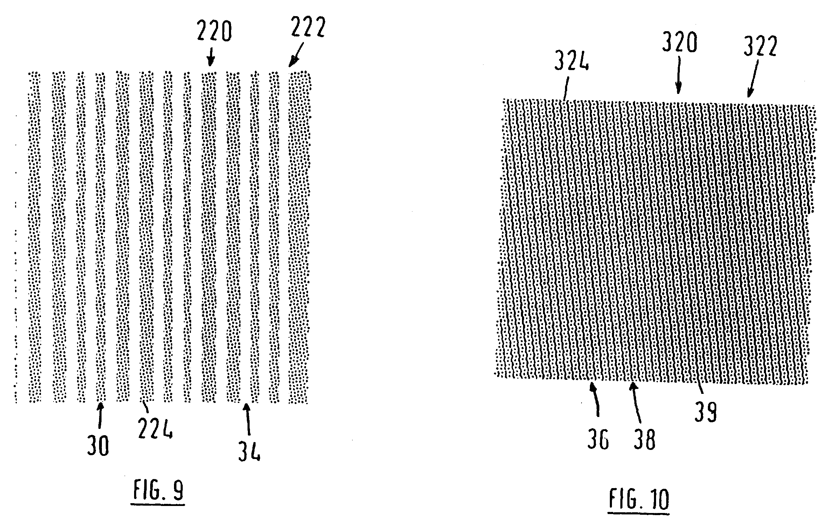

Referring now to Figure 9 of the accompanying drawings, there is illustrated a third embodiment of a secondary topsheet 220 having a regularly spaced second pattern 222 of indicia 224 arranged in stripes 30 having clear areas 34 therebetween.

Referring now to Figure 10 of the accompanying drawings, there is illustrated a fourth embodiment of a secondary topsheet 320 having a regularly spaced second pattern 322. The second pattern 322 comprises an irregular column 36 of spaced-apart indicia 324 and an irregular column 38 of marks 39 forming a wavy line.

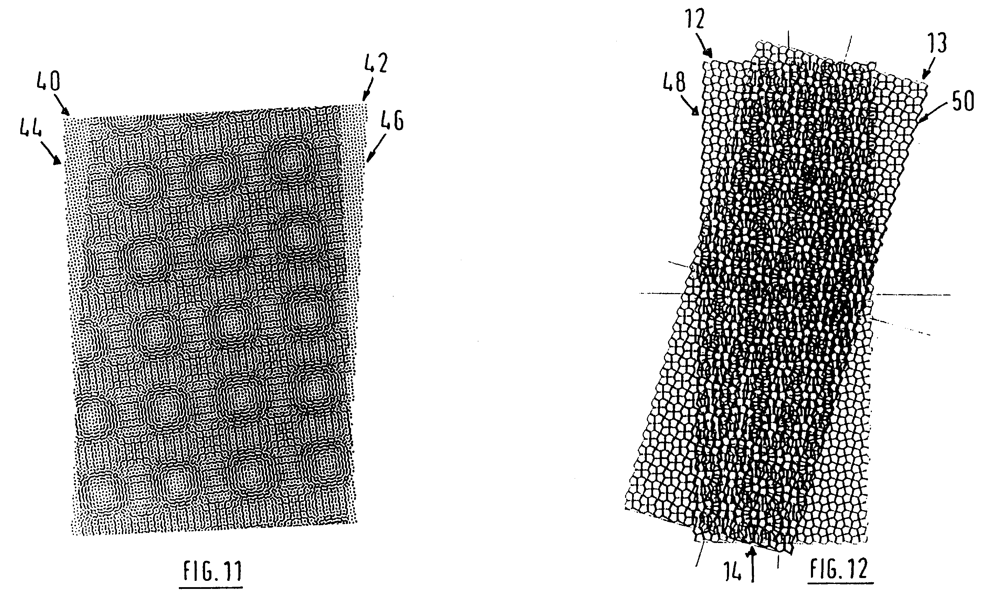

Referring now to Figure 11 of the accompanying drawings, there is illustrated a fifth embodiment of a secondary topsheet 420 having a regularly spaced second pattern 422 of indicia 424. The secondary topsheet 420 comprises a first transparent or translucent secondary top layer 40 and an underlying second secondary top layer 42. The first secondary top layer 40 has indicia 424 arranged in a regularly spaced sixth pattern 44 and the second secondary top layer 42 has indicia 424 arranged in a regularly spaced seventh pattern 46. The second secondary top layer 42 is at a pivot angle of 12.5° relative to the first secondary top layer 40, the composite indicia 424 forming the regularly spaced second pattern 422. Alternatively, a unitary secondary topsheet 420 may be provided with a regularly spaced sixth pattern 44 of indicia 24 and with a regularly spaced seventh pattern 46 of indicia 24 at an angle, for example, 12.5° to the sixth pattern 44, thereby providing the composite indicia 24 forming the regularly spaced second pattern 22. The sixth and seventh patterns 44, 46 may be the same (as shown in Figure 10) or different (not shown), provided that a regularly spaced second pattern 22 is formed.

It will be appreciated that all of the illustrated second patterns 22, 122, 222, 322, 422 have been designed for the primary top layer 12 illustrated in Figures 1 - 7. It will of course be appreciated that any regularly spaced first pattern 14 of apertures 16 may be used on the primary topsheet 10, provided that the second pattern 22 of indicia 24 on the secondary topsheet 20 is designed for the primary topsheet 10 in question.

Referring now to Figure 12 of the accompanying drawings, there is illustrated a second embodiment of a primary topsheet 110, comprising first and second primary top layers 12,13. The first primary top layer 12 has apertures 16 arranged in a regularly spaced fourth pattern 48 and the second primary top layer 13 has apertures 16 arranged in a regularly spaced fifth pattern 50. The first primary top layer 12 is at a pivot angle of 20° relative to the second primary top layer 13, the overlapping apertures 16 forming the regularly spaced first pattern 114. Alternatively, a unitary primary topsheet 110 may be provided with a regularly spaced fourth pattern 48 of apertures 16 and with a regularly spaced fifth pattern 50 of apertures 16 at an angle to the fourth pattern 48. The fourth and fifth patterns 48, 50 may be the same (as shown in Figure 12) or different (not shown), provided that a regularly spaced first pattern 114 is formed.

## Claims

1. An absorbent article comprising a topsheet having a user-facing surface, a backsheet having a garment-facing surface and an absorbent core intermediate the topsheet and the backsheet, characterised in that the topsheet includes a primary topsheet (10; 110) and a secondary topsheet (20; 120; 220; 320; 420); at least some of the primary topsheet (10;110) having apertures (16) arranged in a regularly spaced first pattern (14; 114); the secondary topsheet (20; 120; 220; 320; 420) being in face-to-face relation to the primary topsheet (10; 110); at least some of the secondary topsheet (20; 120; 220; 320; 420) having indicia (24; 124; 224; 324; 424) arranged in a regularly spaced second pattern (22; 122; 222; 322; 422), the indicia (24; 124; 224; 324; 424) being positioned, shaped and dimensioned to be substantially homogeneously visible through the apertures (16) in a first or non-use position, the secondary topsheet (20; 120; 220; 320; 420) being pivoted relative to the primary topsheet (10; 110) by a pivot angle from the first position to a second or in-use position so that the indicia (24; 124; 224; 324; 424) of the secondary topsheet (20; 120; 220; 320; 420) are differentially visible through the apertures (16) of the primary topsheet (10; 110), forming a regularly spaced third pattern (26).

2. An absorbent article according to Claim 1, in which the regularly spaced third pattern (26) overlies the absorbent core and is substantially co-extensive therewith.

3. An absorbent article according to Claim 1 or 2, in which the primary topsheet (10;110) is a three-dimensional formed film.

4. An absorbent article according to Claim 3, in which the pivot angle is not 0°, 90°, 180°, 270° or 360°.

5. An absorbent article according to Claim 4, in which the pivot angle is selected from 0.1°-29.9°, 60.1° - 89.9°, 90.1° - 119.9°, 150.1° - 179.9°, 180.1° - 209.9°, 240.1° - 269.9°, 270.1° - 299.9° or 330.1° - 359.9°, preferably from 5°-20°,70°-85°,95°-110°, 160°- 175°, 185° -200°, 250° - 265°, 275° - 290° or 340° - 355°; even more preferably 7.5° - 15°, 75° - 82.5°, 97.5° - 105°, 165° - 172.5°, 187.5° - 195°, 255° - 262.5°, 277.5° - 285° or 345° - 352.5°.

6. An absorbent article according to any one of Claims 1 - 5, in which the primary topsheet (10; 110) comprises first and second primary top layers (12,13), at least some of each of said first and second primary top layers (12, 13) having apertures (16) arranged in regularly spaced fourth and fifth patterns (48, 50), the regularly spaced first pattern (114) being the overlapping apertures (16) of the regularly spaced fourth and fifth patterns (48,50).

7. An absorbent article according to any one of Claims 1 - 6, in which the secondary topsheet (420) comprises a first transparent or translucent secondary top layer (40), at least some of which having indicia (424) arranged in a regularly spaced sixth pattern (44) and an underlying second secondary top layer (42), at least some of which having indicia (424) arranged in a regularly spaced seventh pattern (46), the regularly spaced second pattern (422) of indicia (424) being the composite indicia (424) of the regularly spaced sixth and seventh patterns (44, 46).

8. An absorbent article according to any one of Claims 1 - 7, in which at least one indicium (24) of the regularly spaced second pattern (22) is positioned, shaped and dimensioned to be substantially homogeneously visible, in the first position, through each aperture (16) of the regularly spaced first pattern (14).

9. An absorbent article according to any one of Claims 1 - 8, in which each indicium (24) of the regularly spaced second pattern (22) is positioned, shaped and dimensioned to be substantially homogeneously visible, in the first position, through an aperture (16) of the regularly spaced first pattern (14).

10. An absorbent article according to Claim 9, in which the indicia (24) forming the regularly spaced second pattern (22) are homogeneously shaped.

11. An method of forming a topsheet for an absorbent article, the method comprising the steps of:-
forming a primary topsheet (10; 110), at least some of said primary topsheet (10; 110) having apertures (16) arranged in a regularly spaced first pattern (14; 114);
forming a secondary topsheet (20; 120; 220; 320; 420), at least some of said secondary topsheet (20; 120; 220; 320; 420) having indicia (24; 124; 224; 324; 424) arranged in a regularly spaced second pattern (22; 122; 222; 322; 422);
pivoting the secondary topsheet (20; 120; 220; 320; 420) relative to the primary topsheet (10; 110) by a pivot angle from a first position, in which the indicia (24; 124; 224; 324; 424) are positioned, shaped and dimensioned to be substantially homogeneously visible through the apertures (16), to a second position, in which the indicia (24; 124; 224; 324; 424) are differentially visible through the apertures (16), forming a regularly spaced third pattern (26);
and fixing the primary and secondary topsheets (10; 110) (20; 120; 220; 320; 420) in the second position.

12. A method of forming an absorbent article comprising a topsheet, a backsheet and an absorbent core intermediate the topsheet and the backsheet, the method comprising the steps of:
forming the topsheet in accordance with the method of Claim 11; and providing the absorbent core and the backsheet in association with the topsheet.
